Europäisches Patentamt

European Patent Office

Office européen des brevets

(19)

(11)     **EP 1 734 028 A1**

(12)                **EUROPEAN PATENT APPLICATION**
                    published in accordance with Art. 158(3) EPC

(43) Date of publication:
**20.12.2006  Bulletin 2006/51**

(51) Int Cl.:
*C07C 27/14* [2006.01]      *C07C 45/32* [2006.01]
*C07C 47/22* [2006.01]      *C07C 51/215* [2006.01]
*C07C 51/25* [2006.01]      *C07C 57/05* [2006.01]

(21) Application number: **04792621.7**

(22) Date of filing: **13.10.2004**

(86) International application number:
**PCT/JP2004/015455**

(87) International publication number:
**WO 2005/100290 (27.10.2005 Gazette 2005/43)**

(84) Designated Contracting States:
**ES IT**

(30) Priority:  **08.04.2004   JP 2004114359**

(71) Applicant: **Mitsubishi Chemical Corporation**
**Tokyo**
**108-0014 (JP)**

(72) Inventors:
• **Ogawa, Yasushi**
**Yokkaichi-shi,**
**Mie 5108530 (JP)**

• **Yada, Shuhei**
**Yokkaichi-shi,**
**Mie 5108530 (JP)**
• **Suzuki, Yoshiro**
**Yokkaichi-shi,**
**Mie 5108530 (JP)**
• **Takasaki, Kenji**
**Yokkaichi-shi,**
**Mie 5108530 (JP)**

(74) Representative: **Vossius & Partner**
**Siebertstrasse 4**
**81675 München (DE)**

(54)    **PROCESS FOR PRODUCING ACROLEIN AND/OR ACRYLIC ACID**

(57)     An object of the present invention is to provide a method of producing acrolein and/or acrylic acid, with which method even when air that contains a lot of floating particles is used as an oxygen source, without suffering from deterioration of catalyst performance owing to coking and so on of the catalyst and inconveniences such as an increase in the pressure difference and clogging of a reaction tube, with a high yield and stably over a long time, acrolein and/or acrylic acid can be produced.

The present invention relates to a method of producing acrolein and/or acrylic acid by subjecting propylene or propane to a gas phase oxidation with an oxygen-containing gas under the presence of an oxidation catalyst, wherein as an oxygen source air from which the floating particles are removed is used.

EP 1 734 028 A1

**Description**

<Technical Field>

The present invention relates to a producing method of acrolein and/or acrylic acid, in more detail, a method in which when propylene or propane is oxidized in a gas phase with an oxygen-containing gas under the presence of an oxidation catalyst to produce acrolein and/or acrylic acid, acrolein and/or acrylic acid is produced by use of air from which floating particles are removed as an oxygen source, without damaging owing to coking and so on the performance of the catalyst, with high yield and with stability over a long period of time.

<Background Art>

It is widely and industrially carried out to produce acrolein and acrylic acid by carrying out catalytic gas phase oxidation of propylene or propane with an oxygen-containing gas. In general, acrylic acid is produced according to a two-stage reaction that includes a first half process in which propylene is subjected to the gas phase oxidation with an oxygen-containing gas to mainly produce acrolein and a second half process in which a reaction gas containing the acrolein is further oxidized to produce acrylic acid.

However, in the first half process where propylene is oxidized with an oxygen-containing gas, other than a principal reaction that generates acrolein, side reactions are caused to generate organic acids such as terephthalic acid and maleic acid, high boiling point compounds and tar-like compounds. Accordingly, a reactor is contaminated with these by-products, in blatant cases, owing to carbide and so on, the coking of the catalyst is caused, an increase in the pressure difference on a reaction tube and the clogging of the reaction tube are caused, resulting in problems in that a normal operation becomes difficult and product quality is deteriorated. Furthermore, when refining means are disposed to remove these by-products, the equipment cost becomes huge to result in a rise in the producing cost; still furthermore, since side reactions due to highly reactive compounds including acrolein are caused, problems are likely to be caused in the deterioration of yield of a target compound and in further deterioration of product quality.

So far, in order to suppress these side reactions, such operations that an exit gas of a reactor of the first half process is rapidly cooled to suppress the side reactions from occurring, or a temperature of an exit gas of a reactor is maintained at a definite temperature or more to suppress the reactor from being contaminated owing to precipitation of high boiling point compounds and tar-like compounds have been carried out (JP-A-49-132007).

Furthermore, with an intention of producing, while suppressing these side reactions from occurring, acrolein and acrylic acid from propylene at a high yield and with stability over a long period of time, a producing method of acrolein and acrylic acid in which a content of particular unsaturated hydrocarbons that are impurities in propylene raw material is made 500 ppm or less (by weight) has been proposed (JP-A-2000-38358).

Since these proposals are insufficient to overcome the problems of an increase in the pressure difference of the reaction tube owing to the coking and so on of the catalyst, further countermeasures have been craved for.

Recently, Japanese enterprises are often establishing overseas factories and frequently producing acrolein and acrylic acid in overseas factories according to the gas phase oxidation of propylene. In such cases, as an oxygen source that is used in the gas phase oxidation of propylene, normally, air at a place where the factory is established is used. It was found that even when reactions are carried out with similar raw materials and under similar conditions, depending on places where the factories are established, there are differences between degrees of an increase in the pressure difference of a reaction tube and between times up to clogging of the reaction tube, and depending upon the place the time becomes unexpectedly shorter.

Incidentally, according to the environmental statistics data base of Environmental Information Center of National Institute for Environmental Studies, environmental criteria of floating particles in Japan is "one day average value of hourly values is 0.10 mg/m$^3$ or less and the hourly value is 0.20 mg/m$^3$ or less". However, it is known that the floating particles are much contained in companies' smoke emission and the Diesel engine vehicle exhaust fumes. In particular, in the neighborhoods of roads full of traffic, in many places, the values are exceeded. For example, according to measurements of 2001 fiscal year, in Ohta Ward of Tokyo, in nearly 100 days in a year, the numerical values were exceeded.

Furthermore, according to report of survey on country-by-country development of environmental information dated October, 1997 due to Japan International Cooperation Agency, it is described that for instance, in China, the air pollution in urban districts is serious, and concentrations of the floating particles in air in the respective cities exceed the environmental criteria; that is, in cities of "The National Environmental Survey Network", annual average values are in the range of 79 to 618 $\mu$g/m$^3$, an average value of urban districts across the country is 309 $\mu$g/m$^3$, and in particular in northern cities these values are higher. Furthermore, it is described that in India, in six large cities of Bombay, Calcutta, Delhi, Ahmedabad, Kanpur and Nagpur, concentrations of the floating particles are, by annual average, more than three times the reference value of WHO and serious air pollution is caused. The floating particles in air are said increasing very much and worldwide concerns are aroused to the environmental problem; thus, in various places, the problems of the air pollution are variously attacked. However, in actuality, the concentrations of the floating particles in air are largely different depending on countries, areas, neighboring equipment and so on.

<Disclosure of the Invention>

The present inventors studied hard reasons why difference in the degree of pressure difference of a reaction tube and difference in the time up to the clogging are caused depending on the aforementioned places where factories are established and found that there are differences in situations of air pollution of places where producing equipment is installed and in particular in amounts of impurities such as the floating particles in air, in heavily polluted places, the time up to the clogging of the reaction tube is likely to be shorter, that is, the coking of the catalyst owing to the floating particles in air cannot be neglected. In this connection, for instance, at an inlet of an air-intake compressor, together with a usually-used rough metal gauze for removing large rubbish, a filter having a fine opening such as 10 $\mu$m or less was disposed to remove the impurities such as the floating particles. Thereby, it was found that the increase of pressure difference of the reaction tube and the clogging are largely reduced, and thereby the present invention was completed.

The aforementioned JP-A-49-132007 and JP-A-2000-38358 neither at all describe nor indicate the use of, as an oxygen source for gas phase oxidization reaction, air from which the floating particles are removed and, furthermore, according to the inventors' experiences, the inventors could not find any description in other literatures of using such an oxygen source in the gas phase oxidation of propylene.

According to the present invention, in a method of producing acrolein and/or acrylic acid owing to the gas phase oxidation of propylene with an oxygen-containing gas under the presence of an oxidation catalyst, a method of producing acrolein and/or acrylic acid by use of, as an oxygen source, air from which the floating particles are removed is provided.

<Best Mode for Carrying Out the Invention>

In the present invention, when propylene or propane is subjected to gas phase oxidation under the presence of an oxidation catalyst by use of an oxygen-containing gas to produce acrolein and/or acrylic acid, as an oxygen source, air from which the floating particles are removed is used.

A method of removing the floating particles from air is not restricted to particular method. For instance, a method of disposing a filter at an inlet of an air-intake, compressor, a method of letting go through water before introducing into a reaction tube, and a method of using air past through an electric dust collector can be cited. In the method of letting go through water, after going through a water bath, air becomes full of water droplets or excessive moisture. The water droplets contain removed floating particles; accordingly, the floating particles are once more introduced into the reaction tube. The excessive moisture increases an amount of gas that is introduced into the compressor. Furthermore, the method of letting go through the electric dust collector demands much cost in installation, maintenance and management of the electric dust collector. Accordingly, as a method of removing the floating particles from air, a method of disposing a filter at an inlet of an air-intake compressor is most economical and large in effect.

When air is used as a raw material or one of auxiliary materials, it is general to dispose an open metal gauze at an inlet of air to remove relatively large rubbish such as wastepaper and leaves. In the invention, together with the metal gauze, a fine filter is disposed to remove the floating particles contained in air, thereby influence on the coking of the oxidation catalyst owing to the floating particles is excluded, resulting in successfully suppressing the increase of the pressure difference applied on the reaction tube and the clogging of the reaction tube.

The fineness (opening) of the opening of the filter, without restricting to particular one, can be properly determined according to particle sizes and concentrations of the floating particles at places where factories are installed. However, with several kinds of filters different in the opening disposed beforehand at an inlet of a compressor, the filter can be selected so that for instance one day average value of hourly values may be, for instance, 0.1 mg/m$^3$ or less that is Japanese environmental standard. As a preferable filter opening, since the floating particles are usually ones of 10 $\mu$m or less, for instance, 10 $\mu$m or less can be illustrated.

The filter, when the openings are clogged, becomes difficult to take in air, and part of trapped floating particles contaminates air past through the filter; accordingly, before that, the filter is necessary to be replaced. A method of determining a replacement timing of the filter is not restricted to particular one. For instance, a method of exchanging a filter when an increment in weight of the filter is always measured and observed and found to be a predetermined value can be preferably adopted. Furthermore, when the clogging of the filter is caused too early with only one kind of filter, in one method, a plurality of filters different in the opening may be combined and used.

Measurement of an increment in weight of a filter can be performed by use of, for instance, a load cell. A value of an increment of weight of the filter that becomes a measure of the replacement timing of the filter can be experimentally determined beforehand.

Furthermore, the pressure difference of the filter may be always measured and observed and when it reaches a predetermined value the filter may be exchanged. As a method of measuring the pressure difference of the filter, for instance, there is a method in which pressure gauges are disposed at inlet and outlet sides of gas (for instance, air) flowing in the filter and difference thereof is taken as the pressure difference. A value of the pressure difference that becomes a measure of the replacement timing of the filter can be experimentally determined beforehand.

The number of filters disposed is not particularly restricted. However, for the cases when frequency of exchange is high owing to serious air pollution or when equipment has to be operated even after expiration of the replacement timing of the filter, two or more filters may be disposed in parallel. Furthermore, a cleaning device of the filter may be disposed in combination.

Impurities such as nitrogen, carbon dioxide, NOx, SOx and moisture contained in air, as far as these are within ranges of amounts normally contained in air, do not particularly adversely affect on the reaction.

Production of acrolein and/or acrylic acid from propylene or propane according to the method of the invention and production of acrylic acid from propylene or propane according to a two-stage reaction according to the invention, except for air from which the floating particles are removed being used as an oxygen source, can be carried out according to a generally applied method.

As the catalyst to be used, catalysts generally used in the production of acrolein and/or acrylic acid from propylene or propane and production of acrylic acid from propylene or propane according to a two-stage reaction can be used respectively. For instance, in the production of acrylic acid owing to the two-stage reaction, as an oxidation catalyst that is used in the first half process where acrolein and/or acrylic acid is generated from propylene or propane, metal oxide catalysts in which molybdenum and bismuth are indispensable ingredients can be cited. As preferable one among these, an oxidation catalyst represented by the following equation (1) can be cited.

$$Mo_aBi_bFe_cA_dD_eE_fG_gO_x \qquad (1)$$

Here, Mo, Bi, Fe and O denote elements that the respective symbols mean; A is at least one kind of element selected from cobalt and nickel; D is at least one kind of element selected from alkali metals, alkaline earth metals and thallium; E is at least one kind of element selected from tungsten, silicon, aluminum, zirconium and titanium; G is at least one kind of element selected from phosphorus, tellurium, antimony, tin, cerium, lead, niobium, manganese, arsenic and zinc; a, b, c, d, e, f, g and x denote atomic ratios of the respective elements; and when a is 12, b is in the range of 0.1 to 10, c is in the range of 0.1 to 20, d is in the range of 2 to 20, e is in the range of 0.001 to 10, f is in the range of 0 to 30, g is in the range of 0 to 4, and x is the number that is determined according to oxidized states of the respective elements.

A shape of the oxidation catalyst, other than a circular, ring-like or spherical shape, may be an indeterminate shape, or as needs arise the oxidation catalyst may be carried on an inactive carrier, or active ingredients may be molded according to an appropriate method. Other than the above ingredients, a molding aid, a reinforcement agent and so on may be added, for instance, various kinds of glass fibers or whiskers can be used.

The oxidation catalyst is not restricted to a single kind. It may be partially diluted with an inactive carrier, or a plurality of kinds of oxidation catalysts different in activity from one another, which are prepared by varying ingredients, preparation methods and sintering conditions may be combined and used.

For a reactor, a multi-tube type fixed bed reactor is generally used. However, a fluid-bed reactor or a moving bed reactor may be used. As a material of the reactor, without particular restriction, carbon steel, stainless steel and so on can be used.

In the invention, when acrylic acid is produced according to the catalytic gas phase reaction, for instance, the two-stage reaction of propylene or propane, after a soluble ingredient such as acrylic acid is recovered or separated by use of a solvent substantially made of water or other solvent from a reaction gas containing acrylic acid that is obtained from the second half process, the whole or part of unreacted propylene or propane can be circulated and used as a raw material gas.

In carrying out the inventive method, after the oxidation catalyst is filled in a multi-tube reactor, to a reaction region thereof, a raw material gas including as a raw material, propylene or propane, as an oxygen source air from which the floating particles are removed and an inert gas that is mixed according to necessity is passed at a temperature in the range of 250 to 450°C, preferably in the range of 280 to 400°C, and at a space velocity in the range of 300 to 5000 hr$^{-1}$, preferably in the range of 700 to 3000 hr$^{-1}$.

<Examples>

In what follows, the present invention will be more specifically explained with Examples.

A conversion ratio of propylene and yields of acrolein and acrylic acid are obtained according to equations below.

$$\text{Conversion ratio of propylene (\%)} = \text{(amount of reacted propylene (mol))/(amount of supplied propylene (mol))} \times 100$$

$$\text{Yield of acrolein (\%)} = \text{(amount of generated acrolein (mol))/(amount of supplied propylene (mol))} \times 100$$

and

$$\text{Yield of acrylic acid (\%)} = \text{(amount of generated acrylic acid (mol))/(amount of supplied propylene (mol))} \times 100$$

Example of Production (preparation of catalyst)

Ninety four parts by weight of antimony paramolybdate were dissolved under heating in 400 parts by weight of pure water, and 7.2 parts by weight of ferric nitrate, 25 parts by weight of cobalt nitrate and 38 parts by weight of nickel nitrate were dissolved in 60 parts by weight of pure water under heating. These solutions were blended under stirring.

In the next place, 0.85 parts by weight of borax and 0.36 parts by weight of potassium nitrate were dissolved in 40 parts by weight of pure water under heating, followed by adding to the above slurry. Subsequently, 64 parts by weight of particulate silica were added therein followed by stirring. In the next place, 58 parts by weight of bismuth subcarbonate in which 0.8 % by weight of Mg is compounded beforehand were added under stirring, the slurry was heated and dried, followed by treating at 300°C for 1 hr in an air atmosphere, further followed by molding obtained particulate solid into tablets having a diameter of 5 mm and a height of 4 mm by use of a molding machine, still further followed by heating at 500°C for 4 hrs, thereby the first half catalyst was obtained.

An obtained catalyst was a Mo-Bi base composite oxide that has a composition ratio of catalyst powder that has a composition of Mo (12) Bi (5) Ni (3) Co (2) Fe (0.4) Na (0.2) Mg (0.4) B (0.2) K (0.1) Si (24) O (x) (composition x of oxygen is a value determined depending on oxidation states of the respective metals).

The obtained catalyst was filled in a stainless tube having an inner diameter of 25 mm and a length of 4200 mm so that a layer length of the catalyst may be 3000 mm and thereby a reaction tube was prepared.

Example 1

The reaction tube prepared in the Example of Production was dipped in a bath of a molten salt (53 % by weight of potassium nitrate, 40 % by weight of sodium nitrite and 7 % by weight of sodium nitrate), and thereto a reaction gas containing propylene in a concentration of 9 % by volume, 76 % by volume of air introduced by letting air that has a concentration of the floating particles of substantially 500 $\mu$g/m$^3$ go through a filter having an opening of 10 $\mu$m and 15 % by volume of a balance gas was supplied at a space velocity of 800 hr$^{-1}$ to carry out a reaction at 330°C. After an initial reaction and after a reaction was continued for 24000 hrs, the conversion ratios of propylene, the yields of acrolein and acrylic acid, and the pressure differences on the reaction tube were as follows.

<Initial reaction>

Conversion ratio of propylene: 97 mol%
Total yield of acrolein and acrylic acid: 92 mol% and
Pressure difference of reaction tube: 6.4 kPa

<After 24000 hrs>

Conversion ratio of propylene: 91 mol%
Total yield of acrolein and acrylic acid: 85.6 mol% and
Pressure difference of the reaction tube: 6.6 kPa
The weight increase of the filter was observed with a load cell and the filter was exchanged every 8000 hrs after the start of the reaction.

Example 2

Except that, in Example 1, in place of a filter having an opening of 10 $\mu$m a filter having an opening of 5 $\mu$m was used, similarly to Example 1 a reaction was carried out. After an initial reaction and after a reaction was continued for 24000 hrs, the conversion ratios of propylene, the yields of acrolein and acrylic acid, and the pressure differences on the reaction tube were as follows.

<Initial reaction>

Conversion ratio of propylene: 97 mol%
Total yields of acrolein and acrylic acid: 92 mol% and
Pressure difference of reaction tube: 6.4 kPa

<After 24000 hrs>

Conversion ratio of propylene: 91 mol%
Total yield of acrolein and acrylic acid: 85.7 mol% and
Pressure difference of reaction tube: 6.5 kPa
The filter was observed of the pressure difference and exchanged at 8000 hrs after the reaction was started.

Comparative Example

Except that in Example 1 without using a filter air is as it is used as an oxygen source, similarly to Example 1, a reaction was carried out. Since when the reaction was continued for 15000 hrs, the pressure difference on the reaction tube became 10 kPa, the reaction was ceased there. After an initial reaction and after a reaction was continued for 15000 hrs, the conversion ratios of propylene, the yields of acrolein and acrylic acid, and the pressure differences on the reaction tube were as follows.

<Initial reaction>

Conversion ratio of propylene: 97 mol%
Total yield of acrolein and acrylic acid: 92 mol% and
Pressure difference of the reaction tube: 6.4 kPa

<After 7000 hrs>

Conversion ratio of propylene: 92 mol%
Total yield of acrolein and acrylic acid: 85.4 mol% and
Pressure difference of the reaction tube: 10 kPa
In comparison with Example 1, in spite of substantially only a half in an operating time, the total yield of acrolein and acrylic acid fell to substantially same value. Since a further yield deterioration could be expected, the catalyst was exchanged.

Reference Example

Except that, in Example 1, in place of air of which concentration of the floating particles is 500 $\mu$g/m$^3$, air of which concentration of the floating particles is 30 $\mu$g/m$^3$ was used and a filter was not used, similarly to Example 1, a reaction was carried out. After an initial reaction and after a reaction was continued for 24000 hrs, the conversion ratios of propylene, the yields of acrolein and acrylic acid, and the pressure differences on the reaction tube were as follows.

<Initial reaction>

Conversion ratio of propylene: 97 mol%
Total yield of acrolein and acrylic acid: 92 mol% and
Pressure difference of reaction tube: 6.4 kPa

<After 24000 hrs>

Conversion ratio of propylene: 91 mol%
Total yield of acrolein and acrylic acid: 85.3 mol% and
Pressure difference of reaction tube: 7.2 kPa
While the invention has been described in detail and with reference to specific embodiments thereof, it will be apparent to one skilled in the art that various changes and modifications can be made therein without departing from spirit and scope thereof.
This application is based on Japanese patent application (Patent Application No. 2004-114359) filed on April 8, 2004, the entire contents thereof being hereby incorporated by reference.

<Industrial Applicability>

According to the present invention, by use of, as an oxygen source, air from which floating particles are removed, even in much polluted areas, inconveniences in that an increase in the pressure difference and the clogging of the reaction tube are relatively shortly caused can be improved, and thereby acrolein and acrylic acid can be stably produced.

**Claims**

1. A method of producing acrolein, acrylic acid or a mixture thereof according to a gas phase oxidation of propylene or propane under the presence of an oxidation catalyst with an oxygen-containing gas, **characterized in that**, as an oxygen source, air from which floating particles are removed is used.

2. The method according to claim 1, **characterized in that** a filter is disposed to a compressor for taking in air to remove the floating particles.

3. The method according to claim 1, **characterized in that** an opening of a filter is determined so that an amount of the floating particles present in air past through the filter is 0.1 mg/m$^3$ or less by one day average value of hourly values.

4. The method according to claim 2, **characterized in that** an opening of the filter is 10 $\mu$m or less.

5. The method according to claim 2, **characterized in that** an increment of weight of the filter is measured to determine an exchange timing of the filter.

6. The method according to claim 2, **characterized in that** pressure difference of the filter is measured to determine an exchange timing of the filter.

**INTERNATIONAL SEARCH REPORT**

| International application No. |
| --- |
| PCT/JP2004/015455 |

A. CLASSIFICATION OF SUBJECT MATTER
Int.Cl⁷ C07C27/14, 45/32, 47/22, 51/215, 51/25, 57/05

According to International Patent Classification (IPC) or to both national classification and IPC

B. FIELDS SEARCHED

Minimum documentation searched (classification system followed by classification symbols)
Int.Cl⁷ C07C27/12-27/14, 45/32-45/39, 47/22, 51/21-51/25, 57/04-57/055

Documentation searched other than minimum documentation to the extent that such documents are included in the fields searched

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| Y | JP 2001-139537 A (Mitsubishi Chemical Corp.),<br>22 May, 2001 (22.05.01),<br>Claims<br>(Family: none) | 1-6 |
| Y | JP 56-113732 A (Nippon Kayaku Co., Ltd.),<br>07 September, 1981 (07.09.81),<br>Claims; page 2, upper left column, line 15 to<br>lower left column, line 6<br>(Family: none) | 1-6 |
| A | JP 2001-220362 A (Nippon Shokubai Co., Ltd.),<br>14 August, 2001 (14.08.01),<br>& US 2001-003783 A1 | 1-6 |

| ☐ Further documents are listed in the continuation of Box C. | ☐ See patent family annex. |
| --- | --- |

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    12 January, 2005 (12.01.05) | Date of mailing of the international search report<br>    01 February, 2005 (01.02.05) |
| --- | --- |
| Name and mailing address of the ISA/<br>    Japanese Patent Office | Authorized officer |
| Facsimile No. | Telephone No. |

Form PCT/ISA/210 (second sheet) (January 2004)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 49132007 A
- JP 2000038358 A

- JP 2004114359 A